**Europäisches Patentamt**

**European Patent Office**

**Office européen des brevets**

(19)

(11) Publication number: **0 234 545**

**A2**

(12) **EUROPEAN PATENT APPLICATION**

(21) Application number: **87102550.8**

(22) Date of filing: **23.02.87**

(51) Int. Cl.³: **C 07 K 15/06**
C 07 K 3/18, C 12 P 21/00
C 07 K 3/28, A 61 K 37/02
A 61 K 35/12
//(C12P21/00, C12R1:91)

(30) Priority: **24.02.86 DE 3605907**

(43) Date of publication of application:
**02.09.87 Bulletin 87/36**

(84) Designated Contracting States:
**AT BE CH DE ES FR GB GR IT LI LU NL SE**

(71) Applicant: **Robapharm AG**
**St. Albanrheinweg 174**
**CH-4006 Basel(CH)**

(72) Inventor: **Sorg, Clemens, Prof. Dr.**
**Alhardstrasse 21**
**D-4400 Münster-Nienberge(DE)**

(74) Representative: **Vossius & Partner**
**Siebertstrasse 4 P.O. Box 86 07 67**
**D-8000 München 86(DE)**

(54) Purified human angiogenic factor, method for its preparation and pharmaceutical preparations.

(57) This invention relates to purified human angiogenic factor (human AF) and its individual proteins, a method for their isolation and also pharmaceutical preparations containing purified human AF and its individual proteins.

FIG. 1

EP 0 234 545 A2

our Ref.: W 247 EP                               23.2.1987

Robapharm AG

St. Albanrheinweg 174, Basle, Switzerland


"Purified Human Angiogenic Factor, Method for its Preparation and Pharmaceutical Preparations"


The invention relates to purified human angiogenic factor (human AF), its individual proteins, methods for their preparation and pharmaceutical preparations containing said factor or said proteins.


## Background of the Invention


The crucial prerequisite for the growth and function of tissues is that they are adequately supplied with blood vessels. Induction of the vascular endothelium to form new blood vessels in regenerative processes such as wound healing and in tumour growth is a complicated multi-stage process which is triggered and controlled by so-called angiogenic factors. These factors induce the capillary endothelium to directed migration, proliferation and formation of a vessel with the associated structures, either individually or in combination (D.H. Ausprunk and J. Folkman, J. Microvasc. Res., Vol. 14, 1977, pp. 53-65. Purified angiogenic factors (AF) and their individual proteins are therefore important for diagnosing inflammatory diseases and tumours, especially for differential diagnosis of endotheliomas, for therapy of inflammatory diseases, tumours, arteriosclerosis and wound healing. They have a role in normal tissue formation and renewal, e.g. in pregnancy and bone growth.


Angiogenic factors belong to the group known as the cytokins. This group includes biologically active, soluble peptides which can be secreted by various cells, for example monocytes, macrophages, endothelial or tumour cells. Other examples of cytokins are interferons ($\alpha$, ß, $\gamma$-interferon), interleukin 1 and 2, macrophage activating factors (MAF), macrophage migration inhibiting factor (MIF) and various growth factors like "epidermal growth factor" (EGF), "fibroblast growth factor" (FGF) and "platelet-derived growth factor" (PDGF). These cytokins stimulate the differentiation, activation and proliferation of various cell types of mesenchymal and epithelial origin.

Various angiogenic factors, though mostly of animal origin, have been described which are able to trigger the phenomenon of new blood vessel formation in a suitable experimental arrangement; R. Auerbach, Lymphokines, Vol. 4, 1981, pp. 69-88. The source for these factors are either tissue extracts (e.g. bovine brain, bovine retina) or tumour tissue or cultured tumour cells.

Little is known about the chemical characteristics and cellular origin of the described angiogenic activities.

It has so far also remained unclear which cells produce angiogenic activities in which physiological or pathological situations. The literature cites tumour cells, lymphocytes and macrophages as possible producers.

Accordingly, although angiogenic factors from human cells are known to be the active principle, they have hitherto only been described in admixture and together with other proteins and biological fluids and, with one exception, they have not been characterized structurally.

In Biochemistry, Vol. 24, 1985, pp. 5486-5494, D.J. Strydom et al. describe the amino acid sequence of an angiogenic factor (angiogenin) isolated from human carcinoma cells (HT29). Angiogenin has a molecular weight of 14,400, an isoelectric point of > 9.5, is biologically active in the rabbit cornea and on the chorioallantoic membrane of the embryonated chicken egg, but does not stimulate growth of cultured human endothelial cells.

## Description of the Invention

The problem underlying the invention is to provide high purity human AF and its individual proteins and a method for isolating same. A further object of the invention is to provide pharmaceutical preparations containing purified human AF or its individual proteins. Hitherto it has not been possible to produce human AF from human cells in pure form.

The invention is based on the following findings:
Human AF is not only secreted by specific monocytes/macrophages, but also by some endothelial and tumour cells, in particular by various tumour cell lines, as can be shown by monoclonal antibodies to human AF. Human AF can be clearly distinguished from MIF, the interferons, MAF and the growth factors such as EGF, FGF and PDGF.

The invention thus relates to purified human angiogenic factor (human AF) and its individual proteins.

Individual proteins of purified human AF are proteins which can be isolated by conventional methods of protein analysis, preferably by preparatory gel filtration, HPLC (high pressure liquid chromatography), anion exchange chromatography, affinity chromatography on heparine-sepharose or adsorption chromatography on hydroxyapatite or combinations of these methods, and they are homogeneous. They are active in standard assays for the detection of angiogenesis.

Human AF consists of two proteins having a molecular weight of about 64,000 (64 kd) and 68,000 (68 kd) and an isoelectric point of about 5.0 to 5.3.

The angiogenin described by D.J. Strydom differs from human AF in all characteristics but the angiogenic activity in the chick embryo test.

Human AF contains only proteins of human origin which exhibit epitopes which are recognized and bound by a monoclonal antibody to human AF. Human AF consists of two individual proteins of a molecular weight of about 64,000 and 68,000 which differ presumably because of their varying glycosylation and possibly further individual proteins or aggregates thereof of higher molecular weight and dissociated products of a lower molecular weight of about 20,000. Human AF is active in standard assays in which the formation of new blood vessels (angiogenesis) is measured on the chorioallantoic membrane of the embryonated chicken egg; test according to Stenzinger et al., Eur. J. Cancer Clin. Oncol., Vol. 19, 1983, pp. 649-686.

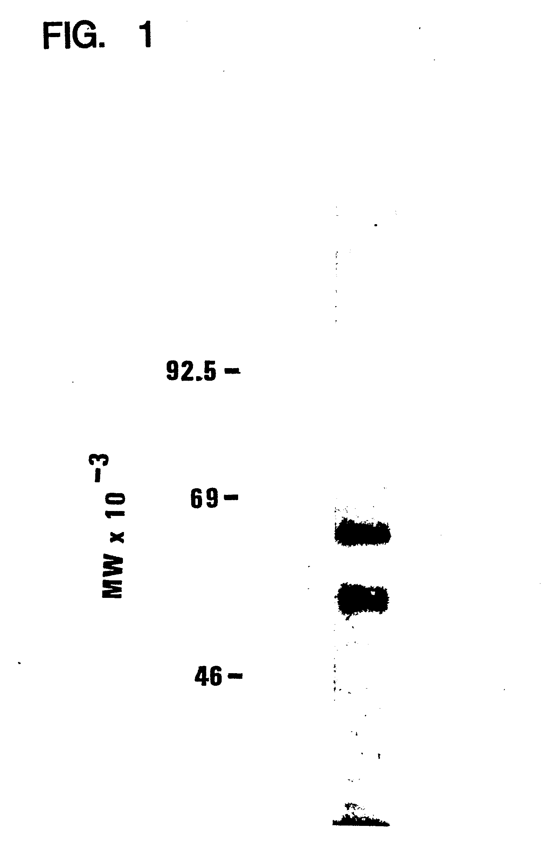

Fig. 1 shows the result of a SDS-polyacrylamide gel electrophoresis (10% acrylamide) of a human AF sample purified by immunoaffinity chromatography: the supernatant of a culture of melanoma cell line A 375/2 was incubated with monoclonal antibodies specific to human AF bound to a carrier. Specifically bound human AF was eluted by applying a voltage of 200 V (10 h in 10 mM Tris borate buffer, pH 8.3) in a membrane trap (Biotrap, Schleicher and Schüll, Dassel). The protein bands were detected by fluorography the supernatant which had been marked with tritium leucin. The position of the marker proteins of defined molecular weight is indicated at the left.

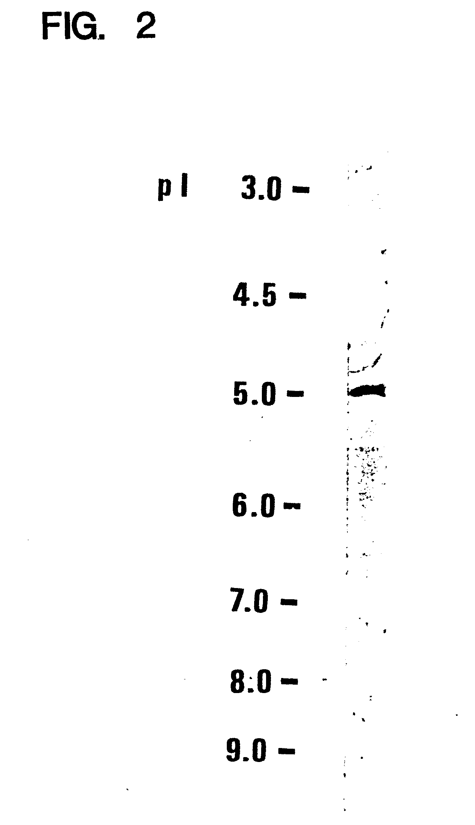

Fig. 2 shows the isoelectric focusing of human AF: the supernatant of a culture of melanoma cell line A 375/2 was separated on a Servalyte commercial gel (Serva, Heidelberg) in a pH gradient of 3 to 10. After focusing, the separated proteins were transferred to nictrocellulose. Human AF was detected by a Western blot with monoclonal antibodies specific to human AF. The isoelectric point (pI) was determined by comparing the positions of the marker proteins.

The invention further relates to a method for preparing purified human AF. The method is characterized by using as starting material a solution containing human AF, preferably a cell culture supernatant or a cell extract of human cells forming human AF. Preferred are established (permanent) human melanoma cells. Suitable human melanoma cells are for example melanoma cell lines A-375/2, Mel-S7, Mel-S13, Mel-2a and MeWO (W. Stenzinger et al., Eur. J. Cancer Clin. Oncol., Vol. 19, 1983, pp. 649-656). A preferred representative of these cells is the melanoma cell line A 375/2.

According to the method of the invention, the solution containing human AF is contacted with monoclonal antibodies specific to human AF bound to a human carrier, unbound proteins, and other foreign substances are removed by washing out. This method is known as immunoaffinity chromatography. Then the human AF bound to the antibody, which has a molecular weight from approx. 64 to 68 kd is eluted and isolated.

Solutions containing human AF, e.g. cell extracts, cell culture supernatants or cell culture filtrates from human cells, are prepared by methods known per se. The solution containing human AF is obtained from the cells and/or the cell culture supernatants, for example by extraction, filtration and/or centrifugation, and if desired stabilized by adding antibiotics and/or protease inhibitors. Such human AF solutions can be contacted directly with monoclonal antibodies specific to human AF bound to a carrier or matrix. Preferably, however, they are first prepurified by ultrafiltration on membranes with exclusion limits of about 1,000 molecular weight and concentrated. If desired these steps are followed by chromatography, e.g. on Polyol-DEAE (Serva, Heidelberg) or by molecular sieving or isoelectric focusing (LKB, Sweden) or chromatofocusing (LKB, Sweden) in accordance with the directions of the manufacturers of separation materials.

According to the method of the invention, human AF is separated from other proteins and foreign substances contained in the solution by immunoaffinity chromatography. For this purpose, the solution containing human AF is contacted by the method known per se with a carrier to which monoclonal antibodies specific to human AF are bound.

Suitable carriers are, for example silicates, cross-linked agarose, dextran or polyacrylamide in suitably functionalized form. Monoclonal antibodies specific to human AF are bound to the carrier in a manner known per se, having previously been activated if necessary. For example a carrier containing activated ester functions, e.g. N-hydroxysuccinimide ester groups, is suspended in an aqueous buffer solution and mixed with a solution of the monoclonal antibody. Subsequently unbound monoclonal antibodies are washed out and unoccupied reactive sites on the carrier are blocked, for instance with a primary amine such as ethanolamine. The carrier bearing the monoclonal antibody is suspended in a suitable aqueous solvent, e.g. a NaCl solution, or a buffer solution such as phosphate-buffered NaCl solution (PBS), $NaHCO_3$ solution or 3-(N-morpholino)-propane sulphonic acid solution, and contacted with the solution containing human AF. For example, the carrier with the monoclonal antibodies is loaded onto a chromatography column and the solution containing human AF is applied and led over the carrier, under pressure if desired. Unbound proteins and other impurities are washed out with aqueous solutions, for example buffer solutions in the pH range from about pH 5 to about pH 9 and/or a 0.1 to 0.3 M, preferably 0.15 M NaCl solution. The human AF bound to the antibodies on the carrier is eluted with suitable aqueous solutions, preferably buffer solutions in the pH range from about pH 2 to about pH 5 such as glycine buffer, or pH gradients of varying compositions or salt solutions, preferably a 3 to 4 Mol $NH_4SCN$ solution. The resulting purified solutions containing human AF are neutralized, if required, and the high purity human AF is isolated from them by methods known per se, for example chromatography on Sephadex®,

electrodialysis, ultrafiltration and/or vacuum centrifugation.

Optionally the human AF is separated into its individual proteins, for example by separating the protein mixture chromatographically into fractions of varying molecular weight by methods known per se. According to a preferred embodiment the purified human AF is separated by preparative sodium dodecyl sulphate-polyacrylamide gel electrophoresis (SDS-PAGE, U.K. Laemmli, Nature, Vol. 227, 1970, p. 680). Homogeneous molecular weight fractions are eluted from the gel by a method known per se and isolated in pure form, e.g. by chromatography on Sephadex®, preferably Sephadex® G-75 or G-100, electrophoretic concentration and/or vacuum centrifugation. Human AF can also be separated by column-chromatographic methods into homogeneous molecular weight fractions and the individual proteins isolated from these. Preferred is a series of separation steps on an anion exchanger column (Mono-Q, HR5-5, Pharmacia, Uppsala), followed by affinity chromatography on heparin-sepharose (No. 17-0467-01; Pharmacia) and finally exchange chromatography on hydroxyapatite (No. 125-0175; Bio-Rad).

The invention furthermore relates to pharmaceutical preparations containing human AF or its individual proteins purified according to the method of this invention.

The pharmaceutical preparations of the present invention are suitable for enteral administration, e.g. nasal, rectal or oral, and preferably for parenteral administration to warm-blooded animals such as man. Depending on the intended route of administration, the pharmaceutical preparations may be in dosage unit form, e.g. in ampoules, vials, suppositories, dragees, capsules or nasal sprays in liquid or solid form.

The amount of therapeutically effective compounds to be administered depends on the condition of the warm-blooded animal, e.g. man, such as body weight, the nature and gravity of the disease and general state of health, as well as on the route of administration, and will be as determined by the physician. The effective dose for human AF and its active individual proteins is in the order of 0.001-10 $\mu$g per kg of body weight daily.

The pharmaceutical preparations according to the invention may contain the conventional inorganic or organic, solid or liquid pharmaceutically acceptable carriers or diluents, possibly together with auxiliary agents. Preferably solutions or suspensions of the active substance are used, particularly isotonic aqueous solutions or suspensions, additionally also lyophilized preparations which are dissolved in water shortly before use. The pharmaceutical preparations may be sterilized and/or contain preservatives, stabilizers, surface-active agents, emulsifiers, solubilizers, viscosity-raising substances, salts for regulating osmotic pressure and/or buffers, as well as other proteins, for example human serum albumin or human blood plasma preparations.

The examples which follow illustrate the invention.

## Example 1

### Production of Crude Human AF

To produce human AF human melanoma cell line A-375/2 was incubated in a density of about 4 x 10$^6$ cells per culture dish (Nunclon T 600, catalogue No. 166, 508) in 100 ml of Minimum Essential Medium (MEM) with Earle's salts (Gibco), 15% foetal calf serum (Biochrom, Berlin) being added. The cells were incubated for 24 hours at 37ºC and then washed three times each with 100 ml of MEM Earle's serum-free and incubated for a further 72 hours at 37ºC with MEM Earle's, adding penicillin-streptomycin, glutamine, non-essential amino acids, sodium pyruvate and HEPES, in each case in a 1% concentration. After 72 hours the supernatants were removed and the cells were extracted by repeated freezing and thawing at -80ºC. The extracts and the supernatants were united and centrifuged for 30 minutes at 250 x g.

## Example 2

### Identification of Human AF (CAM Test)

8-day old embryonated chicken eggs were drilled at their blunt end and the drill hole was covered with a stable film. After a further 1 - 2 days a window 1 x 1 cm in size was drilled at the pointed end of the egg. The shell was removed with sterile plastic tweezers and the outer edge of the window was coated with silicon fat. The window was hermetically sealed with a sterile glass cover. After a further 2 days (day 10) the test substance which was spotted onto a glass fibre filter plate with a diameter of 0.5 cm (60 µl/plate), was placed on the chorioallantoic membrane (CAM). After incubating for 3 - 4 days in a humidified incubator (37ºC) the glass covers were removed, the embryos were killed by immersing them in a 4% formaldehyde solution for 30 min, and the CAM was excised together with the filter plates and placed in a petri dish with 4% formaldehyde solution. A positive reaction was indicated by a ring of new capillary vessels below or around the plate.

Example 3

Preparation of Hybridoma Cells and Monoclonal Antibodies Specific to Human AF

3.1 Human AF Isolation

150 ml of the material isolated in accordance with Example 1 from a serum-free culture supernatant of cell line A-375/2 were concentrated to 1 ml using a CX-10 immersion filter (Millipore, Catalogue No. PTB C11K25). This concentrate was fractionated on a TSK-125 sieve column (Bio-Rad, Munich) by HPLC. The fractions containing human AF were identified by means of a monoclonal antibody after aliquots had been spotted onto nitrocellulose. Positive fractions were pooled and once more concentrated to 2 ml. These preparations contained approx. 40 $\mu$g protein/ml. This material was utilized for coupling to erythrocytes as in Example 3.2.

3.2 Conjugation of Human AF-Containing Protein Fractions to Sheep Erythrocytes (SRBC)

2.5 ml of packed sheep erythrocytes (sheep red blood cells, (SRBC), Behringwerke) were suspended in 20 ml of phosphate-buffered saline solution (PBS). 100 $\mu$l of this SRBC suspension were incubated with 900 $\mu$l of a glutardialdehyde solution in PBS for 5 min at room temperature, so that the final concentration of glutardialdehyde was 0.05%. The thus pretreated SRBC were washed twice with ice cold distilled water, centrifuged off at 2000 x g, and then incubated for 1 hr at 20°C with 300 $\mu$l of the fractions containing human AF from Example 3.1. This suspension was employed for immunization in Example 3.3.

## 3.3 Immunization

BALB/c mice were immunized with 3 injections each of 0.5 ml of the SRBC suspension coupled with human AF from Example 3.2 together with 0.5 ml of complete Freund's adjuvant on day 0, 7 and 10. Half of the injected quantity was given intraperitoneally (i.p.), and the other half subcutaneously (s.c.) in four portions. Two further ('booster') injections each of 0.5 ml of conjugate suspension without adjuvant were administered i.p. on day 13 and 14. The spleen of the treated mice was removed on day 18.

## 3.4 Cell Fusion

In accordance with the method of Köhler and Milstein (G. Köhler and C. Milstein, Nature, Vol. 256, 1975, pp. 495-497) about $10^8$ spleen lymphocytes were mixed with about $3.3 \times 10^7$ mouse myeloma cells P3-X63-Ag8.653 (J.F. Kearney et al., J. Immunol., Vol. 123, 1979, pp. 1548-1550) in 1.5 ml of a solution of 35% polyethylene glycol 4000 (Merck, Darmstadt) and 9.7% dimethyl sulphoxide in Dulbecco's modified Eagle's medium. After fusion the cells were plated out in 600 wells of Falcon 3040 96-well plates and cultivated in Littlefield's HAT medium (J.W. Litttlefield, Science, Vol. 145, 1964, pp. 709-710) together with about $10^4$ bone marrow macrophages per well as feeder cells (Neumann and Sorg, Eur. J. Immunol., Vol. 10, 1980, pp. 834-840). After 10 days in HAT medium the cells were cultivated further in RPMI 1640 HT medium.

## 3.5 Examining the Hybridoma Cells for Antibody Specifity

## 3.5.1 Gammaglobulin Identification

The supernatants of the hybridoma cell cultures were examined in an ELISA test in which a second antibody from rabbit conjugated with peroxidase which recognizes and binds mouse gammaglobulin was employed (Suter et al., J. Immunol. Meth., Vol. 39, 1980, pp. 407-411).

3.5.2 Specificity for Proteins in the Desired Molecular Weight Range

Syngenic mouse erythrocytes (about 1 x $10^6$ erythrocytes in 100 $\mu$l of PBS per well) were applied to 96-well plates (Dynatech Microtiter) coated with poly-L-lysine (25 mg/ml) and incubated overnight at 4$^o$C. Non-bound erythrocytes were removed by washing several times with PBS. The thus bound erythrocytes were, as outlined in Example 3.2, fixed in glutardialdehyde, washed and then incubated with the fractions containing human AF. Non-bound protein was removed by washing several times and the plates were stored in PBS containing 0.1% $NaN_3$. The plates were incubated with culture supernatant from the hybridoma cells, then with a rabbit anti-mouse immunoglobulin second antibody coupled with alkaline phosphatase, and developed with p-nitrophenylphosphate in 2-amino-2-ethyl-1,3-propanediol buffer (Serva) (Suter et al., J. Immunol. Meth., Vol. 39, 1980, pp. 407-411). The liberated p-nitrophenol was identified photometrically at 405 nm. The ELISA test described recognized monoclonal antibodies which bind to the erythrocyte-coupled proteins of the molecular weight fractions containing human AF of Example 3.2.

3.5.3 Specificity for Human AF

Gammaglobulins from the culture supernatants of the clones identified as in Example 3.5.2 were precipitated with ammonium sulphate at 50% saturation, the precipitates were taken up in PBS and coupled according to a direction from the manufacturer to Affi-Gel 10 (Bio-Rad). The gammaglobulins thus immobilized were incubated with supernatants containing human AF overnight at 4$^o$C and then centrifuged at 2000 x g and 4$^o$C for 10 min in an IEC centrifuge. The supernatant solution was then examined for its residual human AF in the CAM test according to Example 2. In this manner three positive cell clones were obtained. One of them was designated as 5F4.

3.5.4 Isolation and Purification of the Monoclonal Antibodies

BALB/c mice were pretreated intraperitoneally with 0.4 ml of Pristan® (Carl Roth Karlsruhe). After a week about 2 to 5 x 10⁶ cloned hybridoma cells were injected i.p. Ascites fluid was taken from each mouse repeatedly and frozen at -80°C. The fluid collected was thawed and centrifuged for 30 min at 4°C at 30000 x g. The fat was aspirated and a saturated ammonium sulphate solution was slowly added dropwise to the remaining debris-free supernatant with stirring at 0°C until a 50% concentration was reached. The crude immunoglobulin fraction precipitated in this manner was chromatographed with 0.1 mol Tris HCl (pH 8.2) over DEAE Affi-Gel Blue (Bio-Rad) as directed by the manufacturer, the active fractions were united and concentrated with Amicon XM50 filter (Amicon).

Example 4

Preparation of an Antibody Column

Affi-Gel 10 (Bio-Rad) was washed with cold distilled water and coupling buffer (PBS, 0.15 M, pH 7.2) as directed by the manufacturer. A 50% suspension of the gel in coupling buffer (1 ml) was transferred to a plastic tube, mixed with the same amount of purified antibody solution (20 mg of monoclonal antibody) and rotated for 4 hrs at room temperature. Thereafter the gel was washed with coupling buffer. To block the free sites that were still active the gel was treated with 0.1 ml of 1 M ethanolamine-HCl (pH 8.0) per ml of gel for 2 hrs at room temperature, then washed with PBS containing 10 mM sodium azide per ml of gel and kept there at 4°C. The degree of coupling was determined by measuring the extinction at 280 nm and totalled 12 to 30 mg of monoclonal antibodies per ml of gel.

Example 5

Isolation and Purification of Human AF Proteins

5.1 Production of Human AF

Culture supernatants of cell line A-375/2 were prepared by the method described in Example 1. Approx. 12 liter of serum-free culture supernatants were concentrated using a Pellicon cassette system (Millipore) with a membrane having a molecular weight exclusion of $>$10,000 to about 160 ml and dialyzed against 0.01 M PBS.

5.2 Immunoaffinity Chromatography

20 ml of the concentrated crude human AF were repeatedly pumped through an antibody column (Example 4) containing 4 ml of gel with a coupling degree of 12 mg of antibody per millilitre of gel at 4°C at a flow rate of 10 ml per hour. Unspecifically bound proteins and other accompanying substances were washed out with 100 ml of PBS, to which 0.1% octylglycoside had been addedd, and finally eluted from the column with 10 ml of 0.1 mol aqueous NaCl solution at a flow rate of 15 ml per hour. The specifically bound human AF proteins were eluted with an aqueous solution of 0.1 mol glycine hydrochloride/0.1 mol NaCl, pH 2.6. The elution was followed by automatic measurement of the absorption at 280 nm (Uvicord S, LKB Instruments). The fractions containing protein were united and neutralized by adding 1 mol Tris solution and assayed for biological activity as in Example 2. This material was angiogenic.

5.3 Analysis of Human AF Proteins by SDS Polyacrylamide Gel Electrophoresis (SDS-PAGE)

One aliquot (about 2%) of the dialyzed concentrate (Example 5.1) was subjected to electrophoresis according to the method of Laemmli (U.K. Laemmli, Nature, Vol. 227, 1970, pp. 680-685) on 12.5% polyacrylamide flat gel. The protein bands were visualized by staining them with Comassie brilliant blue (Fluka) or the silver staining method of C.R. Merril et al. (Anal. Biochem., Vol. 110, 1981, pp. 201-207). The material eluted from the antibody column then contained proteins of molecular weight of about 64,000 (64 kd) and approx. 68,000 (68 kd).

In another batch an aliquot (approx. 2%) of the dialyzed concentrate of Example 5.1 was subjected to electrophoresis by the foregoing method of Laemmli on 15% polyacrylamide flat gel and then transferred by electroelution onto a membrane (Biotrap, Schleicher and Schüll) as indicated by Towbin et al., Proc. Nat. Acad. Sci., Vol. 76, 1979, pp. 4350-4354 and the 1983 procedure laid down by Bio-Rad Laboratories for the Trans-Blot[R] apparatus and stained with a monoclonal antibody specific to human AF proteins in the indirect immune peroxidase procedure. On the blot two bands of molecular weight approx. 64,000 (64 kd) and approx. 68,000 (68 kd) were visible.

5.4   Preparative Separation and Isolation of Human AF Proteins Using HPLC Gelfiltration

The material isolated in Example 5.2 was separated on a TSK 125 molecular sieve (Bio-Rad). The eluting agent used was PBS, pH 7.2 with a flow rate of 0.5 ml/min. At $OD_{280}$ the material gave two peaks of $> 80,000$ and approx. 60,000 - 70,000. Pools of both molecular weight ranges were dialyzed against 50 mM ammonium bicarbonate solution and then lyophilized, taken up in bidistilled water after drying and lyophilized again. An aliquot of each of these preparations was tested once for human AF in the CAM test (Example 2).

5.5  Preparative Separation and Isolation of Human AF Proteins by Ion Exchange HPLC and Immunoaffinity Chromatography

10 ml of the material obtained as in Example 2 were loaded at room temperature on a Mono-Q anion exchanger column (HR5/5, No. 170546-01; Pharmacia) and eluted with a linear saline gradient. Mobile phase A) was 0.01 M PBS, pH 7.2 and mobile phase B) was 1 M NaCl plus 0.1 M PBS, pH 7.2.  An aliquot of each fraction was spotted onto nitrocellulose and stained with a monoclonal antibody specific to human AF according to the method of Towbin et al. (see Example 5.3).  Positive fractions in the range of 0.4 M NaCl were pooled and concentrated to 1 ml using the Millipore Immersible CX10 ultrafilter and dialyzed against 0.1 M PBS and tested for biological activity according to Example 2.  This material was angiogenic.

In a further step this material was subjected to affinity chromatography on heparin-sepharose CL-6B (No. 17-0467-01; Pharmacia). For this the material was dialyzed against 0.01 M PBS and loaded onto the heparin column (40 ml gel bed), which had previously been equilibrated with mobile phase (0.01 M Tris + 0.1 M NaCl; pH 7.0).  Elution over a linear gradient with mobile phase B) then followed (0.01 M Tris, 2.2 M NaCl, pH 7.2).  The fractions were again spotted onto nitrocellulose and detected with the monoclonal antibody specific to human AF proteins.  Monoclonal antibodies specific to human AF were added to the human AF proteins. Monoclonal antibodies bound to the antigen (human AF) were detected with a peroxidase-labelled antiserum (goat) which is directed against mouse immunoglobulins by adding Substral buffer solution (50 mg diamino benzidine in 100 ml PBS and 40 $\mu$l $H_2O_2$).  The intensity of the colour is directly proportional to the existing amount of antigen (human AF) spotted onto nitrocellulose.  Positive fractions were pooled, dialyzed against 0.15 M PBS and tested for biological activity as in Example 2.  This material, which was eluted at 0.5 M NaCl, was angiogenic.

In a third step this material was purified on hydroxyapatite (No. 125-0175; Bio-Rad). After dialysis against 1/10 concentrated buffer (mobile phase A): 0.01 M $Na_2HPO_4$ + 0.01 mM $CaCl_2$; pH 6.8) the material was loaded onto a hydroxyapatite column (gel bed 4.8 ml) equilibrated with mobile phase A), and eluted using a linear gradient with mobile phase B) (0.5 M $Na_2HPO_4$ and 0.01 mM $CaCl_2$; pH 6.8). Aliquots of the fractions were again spotted onto nitrocellulose and stained with the monoclonal antibody specific to human AF proteins. Positive fractions which eluted at 0.5 M NaCl were pooled, dialyzed against 0.15 M PBS and assayed for biological activity as in Example 2. This material too was angiogenic.

Separation of this material on a molecular sieve column (TSK-125) with PBS as mobile phase gave a homogeneous peak at a molecular weight between 60,000 and 70,000. The separation of said material by SDS-PAGE according to the method of Laemmli in Example 5.3 on 15% polyacrylamide flat gel and subsequent transfer onto nitrocellulose and staining with the monoclonal antibody specific to human AF protein gave two bands in the molecular weight range 64,000 and 68,000 and one band at 20,000.

5.6   Determining the Isoelectric Point of Human AF

50 µg of protein from the material obtained from culture supernatants containing human AF in Example 5.1 were dialyzed against 50 mM ammonium bicarbonate solution, lyophilized, taken up in 100 µl of bidistilled water and isoelectrically focused on a commercial gel (Precote, Serva) in the pH range 3 - 10. The gel was blotted onto nitrocellulose and stained with a monoclonal antibody specific to human AF proteins according to the method of Towbin et al. (see Example 5.3). A single band in pH range 5.0 - 5.3 was discernible.

Patent Claims

1. Purified human angiogenic factor (human AF) and its individual proteins.

2. The purified human AF according to claim 1, characterized by containing only proteins of human origin and by containing epitopes that are recognized and bound by antibodies to human AF and is active in a standard assay in which the new formation of capillary vessels is measured.

3. The purified human AF according to claim 1, characterized in that it comprises at least 2 molecules in the molecular weight range of about 64,000 (64 kd) and 68,000 (68 kd) and in addition that it can decompose by dissociation into biologically active proteins of molecular weight about 20,000.

4. The purified human AF according to claim 3, characterized in that the molecules of molecular weights of about 64,000 and 68,000 possess an isoelectric point of about 5.0 to 5.3.

5. The purified human AF according to claim 3 and 4, characterized in that it posesses affinity to heparin-sepharose and can be isolated at a salt concentration of about 0.5 M NaCl.

6. Method for preparing purified human AF, characterized by contacting a solution containing human AF, with monoclonal antibodies specific to human AF bound to a carrier, removing unbound proteins and other foreign substances, separating and isolating the human AF bound to the antibodies, the human AF having a molecular weight in the range from about 64,000 (64 kd) to 68,000 (68 kd).

7. The method according to claim 6, wherein the solution containing human AF is a cell culture supernatant or a cell extract of established (permanent) human melanoma cells.

8.   The method according to claim 6, wherein the solution containing human AF is subjected to ultrafiltration and concentrated before immunoaffinity chromatography.

9.   The method according to claim 6, wherein the purified human AF is separated into its individual proteins of molecular weight about 64,000 (64 kd) and 68,000 (68 kd) by preparative sodium dodecyl sulphate-polyacrylamide gel electrophoresis.

10.   The method according to claim 6, wherein the purified human AF is separated into its individual proteins by preparative gel filtration, HPLC (high pressure liquid chromatography), anion exchange chromatography, affinity chromatography on heparin-sepharose, adsorption chromatography on hydroxyapatite or a combination of these methods.

11.   Pharmaceutical preparations for the treatment of vascular diseases or diseases due to inadequate vascular supply or damage to blood vessels which contain purified human AF or its individual proteins in accordance with claim 1 together with a pharmaceutically acceptable carrier.

12.   Pharmaceutical preparations according to claim 11 which contain purified human AF or its individual proteins obtained in accordance with claims 6 to 10.

FIG. 1

FIG. 2

pI    3.0 –

      4.5 –

      5.0 –

      6.0 –

      7.0 –

      8.0 –

      9.0 –